# EUROPEAN PATENT APPLICATION

(11) **EP 4 740 902 A1**
(43) Date of publication of application: **13.05.2026**
(21) Application number: 24836370.7
(22) Date of filing: 05.07.2024
(51) Int. Cl.: A61C 13/00, A61C 13/34, A61C 13/08, A61C 9/00, A61C 19/04, A61B 5/00, G06F 3/14, G06T 17/00, G06T 1/00, G06F 17/00

(54) **APPARATUS AND METHOD FOR GENERATION AND REAL-TIME MODIFICATION OF MARGIN LINE**

(30) Priority: 06.07.2023 KR 20230087608; 04.07.2024 KR 20240087912
(71) Applicant: Medit Corp., Seoul 07207 (KR)
(72) Inventor: LEE, Sunghoon, Seoul 07207 (KR)
(74) Representative: MFG Patentanwälte Meyer-Wildhagen Meggle-Freund Gerhard PartG mbB
(86) International application number: PCT/KR2024/009551
(87) International publication number: WO 2025/009923

(57) **Abstract**

A method performed by an electronic apparatus according to one embodiment of the present disclosure may include identifying an initial point on a three-dimensional oral cavity model, determining a first search direction from the initial point, based on curvature information about the three-dimensional oral cavity model, sequentially determining multiple first search points on the three-dimensional oral cavity model, based on the curvature information and the first search direction, each of the multiple first search points being determined based on the curvature information and one or more first search points determined immediately before, and generating a margin line indicating a closed path on the three-dimensional oral cavity model, based on the multiple first search points.

## Description

### TECHNICAL FIELD

The technical idea of the present disclosure relates to an apparatus and method for generating and modifying a margin line in real time and, more particularly, to an apparatus and method for generating and modifying a margin line of a tooth in real time on the basis of a three-dimensional (3D) image of an oral cavity received from a 3D scanner.

### BACKGROUND

In general, in order to acquire oral cavity information of a patient, a 3D scanner that is inserted into the patient's oral cavity and acquires an image of the oral cavity may be used. For example, a doctor may insert a 3D scanner into the patient's oral cavity, scan the patient's teeth, gum, and/or soft tissue, thereby acquiring multiple two-dimensional images of the patient's oral cavity, and by applying a 3D modeling technique, a 3D image of the patient's oral cavity may be constructed using the two-dimensional (2D) images of the patient's oral cavity.

Meanwhile, artificial structures such as dental prostheses may be used as a method for treating a patient's teeth. One of the important preprocessing operations in the process of creating a prosthesis is configuring a margin line. The margin line is used to define the boundary between a part exposed to the outside (outer surface) and the remaining part (inner surface) when the prosthesis is inserted into the patient's oral cavity, and may have a decisive effect on whether the prosthesis is well contacted and supported by the patient's oral structure. In particular, prostheses such as inlays and veneers may have many curves, and therefore, frequent operations to precisely modify the margin line may require a lot of time. Technologies for automatically generating margin lines and modifying margin lines have not been sufficiently provided.

### DISCLOSURE

### TECHNICAL PROBLEM

The technical idea of the present disclosure is to provide an apparatus and method for generating a margin line of a tooth and modifying the margin line in real time.

### TECHNICAL SOLUTION

According to an aspect of the technical idea of the present disclosure, a method performed by an electronic apparatus may include an operation of identifying an initial point on a 3D oral cavity model, an operation of determining a first search direction from the initial point, based on curvature information about the 3D oral cavity model, an operation of sequentially determining multiple first search points on the 3D oral cavity model, based on the curvature information and the first search direction, each of the multiple first search points being determined based on the curvature information and at least one most recently determined first search point, and an operation of generating a margin line indicating a closed path on the 3D oral cavity model, based on the multiple first search points.

An electronic apparatus according to an aspect of the technical idea of the present disclosure may include one or more processors, and one or more memories storing instructions executed by the one or more processors, wherein the one or more processors may be configured to, when the instructions are executed by the one or more processors, execute one of the methods according to an aspect of the technical idea of the present disclosure.

A non-transitory computer-readable recording medium according to an aspect of the technical idea of the present disclosure may record instructions that, when executed by one or more processors, cause the one or more processors to perform operations, wherein the instructions may be configured to cause the one or more processors to perform one of the methods according to an aspect of the technical idea of the present disclosure.

### ADVANTAGEOUS EFFECTS

According to an exemplary embodiment of the present disclosure, an apparatus and method may automatically and quickly generate a margin line of a tooth on the basis of a 3D image of the oral cavity.

According to an exemplary embodiment of the present disclosure, an apparatus and method may allow a user to modify margin lines in real time by performing a minimum of operation while viewing incorrectly generated areas during margin line generation.

According to an exemplary embodiment of the present disclosure, an apparatus and method may quickly and efficiently perform a margin line operation for generating a prosthesis through automatic generation and real-time modification of margin lines.

The effects acquirable from the exemplary embodiments of the present disclosure are not limited to the effects mentioned above, and other effects not mentioned may be clearly derived and understood by a person having ordinary skill in the art to which the exemplary embodiments of the present disclosure belong from the following description. That is, unintended effects resulting from practicing the exemplary embodiments of the present disclosure may also be derived by a person having ordinary skill in the art from the exemplary embodiments of the present disclosure.

### DESCRIPTION OF DRAWINGS

FIG. 1 is a diagram illustrating a system for acquiring scan data by using a 3D scanner 200 according to an exemplary embodiment of the present disclosure.
FIG. 2 is a block diagram of an electronic apparatus 100 and a 3D scanner 200 according to an exemplary embodiment of the present disclosure.
FIG. 3 is a perspective diagram of a 3D scanner 200 according to an exemplary embodiment of the present disclosure.
FIGS. 4A and 4B are diagrams illustrating maxillary scan data 301, mandibular scan data 302, and bimaxillary scan data 300 of an oral cavity structure according to an exemplary embodiment of the present disclosure.
FIG. 5 is a diagram illustrating a process of identifying an initial point for margin line generation according to an exemplary embodiment of the present disclosure.
FIG. 6 is a diagram illustrating curvature information for a 3D oral cavity model according to an exemplary embodiment of the present disclosure.
FIGS. 7A, 7B, and 7C are diagrams illustrating multiple first search points, multiple second search points, and an initial closed path according to an exemplary embodiment of the present disclosure.
FIGS. 8A and 8B are diagrams illustrating generation of a margin line according to an exemplary embodiment of the present disclosure.
FIG. 9 is a flowchart illustrating a method for generating a margin line according to an exemplary embodiment of the present disclosure.
FIG. 10 is a flowchart illustrating a method for sequentially determining search points according to an exemplary embodiment of the present disclosure.
FIG. 11 is a flowchart illustrating a method for generating a margin line according to an exemplary embodiment of the present disclosure.
FIG. 12 is a flowchart illustrating a method for generating a margin line indicating a closed path according to an exemplary embodiment of the present disclosure.
FIGS. 13A, 13B, 13C, and 13D are diagrams illustrating a method for modifying a margin line according to an exemplary embodiment of the present disclosure.
FIG. 14 is a flowchart illustrating a method for modifying a margin line according to an exemplary embodiment of the present disclosure.
FIG. 15 is a flowchart illustrating a method for modifying a margin line on the basis of a target line segment according to an exemplary embodiment of the present disclosure.

### MODE FOR INVENTION

The various embodiments described in the present disclosure are illustrated for the purpose of clarifying the technical ideas of the disclosure and are not intended to limit the disclosure to any particular embodiment. The technical ideas of the present disclosure include various modifications, equivalents, and alternatives of each embodiment of the present disclosure and include embodiments optionally combined from all or part of each embodiment. Furthermore, the scope of the technical ideas of the present disclosure is not limited to the various embodiments set forth below or to the specific description thereof.

Terms used in the present disclosure, including all technical and scientific terms, are intended to have the meanings commonly understood by those of ordinary skill in the art to which the present disclosure belongs, unless otherwise defined.

Expressions such as "comprises", "include", "may include", "provided", "may be provided", "has", "may have", and the like used in the present disclosure imply the presence of the subject feature (e.g., a function, an operation, or a component) and do not exclude the presence of other additional features. That is, the expressions should be understood as openended terms that imply the possibility of including other embodiments.

The singular form used in the present disclosure may include a meaning of a plurality, unless otherwise mentioned, and the same is applied to a singular expression stated in the claims.

Expressions such as "first", "second", and the like used in the present disclosure distinguish one object from another in referring to multiple objects, unless otherwise indicated in the context, and are not intended to limit the order or importance of corresponding objects.

In the present disclosure, expressions such as "A, B, and C," "A, B, or C," "A, B, and/or C," "at least one of A, B, and C," "at least one of A, B, or C," "at least one of A, B, and/or C," "at least one selected from A, B, and C," "at least one selected from A, B, or C," and "at least one selected from A, B, and/or C" may refer to each of enumerated items or any possible combination of the enumerated items. For example, "at least one selected from A and B" may refer to (1) A, (2) at least one of A, (3) B, (4) at least one of B, (5) at least one of A and at least one of B, (6) at least one of A and B, (7) at least one of B and A, (8) both A and B.

The expression "based on" or "according to" used in the present disclosure is used to describe one or more factors that influence a decision, an activity of judgment, or an operation described in a phrase or sentence including the relevant expression, and this expression does not exclude an additional factor influencing the decision, the activity of determination, or the operation.

In the present disclosure, expressions such as a certain component (e.g., a first component) being "connected to" or "coupled with" any other component (e.g., a second component) may refer to the certain component being directly connected or coupled to the other component, as well as being connected or coupled through another intervening component (e.g., a third component).

In the present disclosure, the expression "configured to" have meanings such as, depending on the context, "set to", "having an ability to", "changed to", "made to", "to do", and "able to". The expression is not limited to the meaning of "specifically designed in hardware", and for example, a processor configured to perform a specific operation may mean a special purpose computer structured through programming to perform the specific operation.

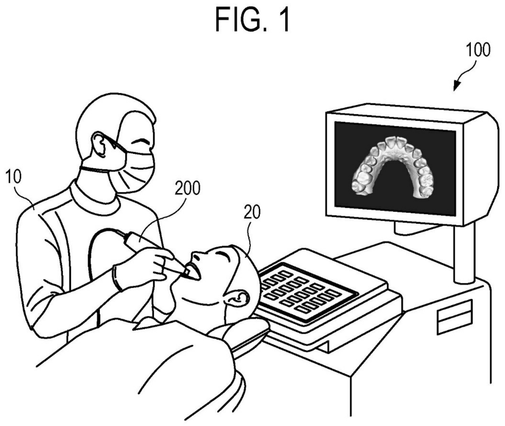

FIG. 1 is a diagram illustrating a system for acquiring scan data by using a 3D scanner 200 according to an exemplary embodiment of the present disclosure.

According to an embodiment, the 3-dimensional (3D) scanner 200 may be a dental medical device for acquiring scan data of a surface of a target subject 20. Here, the surface of the target object 20 may be an inner surface of the oral cavity of the target subject 20.

For example, the 3D scanner 200 may include an intraoral scanner. For example, a user 10 (e.g., a dentist or a dental hygienist) may acquire scan data on the intraoral structure of the subject 20 (e.g., a patient) by using the 3D scanner 200.

For example, the user 10 may acquire an image of the intraoral structure of the subject 20 from a diagnosis model (e.g., a plaster model or an impression model) molded after the shape of the intraoral structure of the subject 20.

Hereinafter, for convenience of explanation, it is described that scan data for the intraoral structure of the subject 20 is acquired by scanning the intraoral structure of the subject 20, but the present disclosure is not limited thereto, and it is also possible to acquire scan data for other parts of the subject 20.

For example, the 3D scanner 200 may have a shape capable of being inserted in and drawn out of the oral cavity, and may be a handheld scanner in which the user 10 may freely adjust a scanning distance and a scanning angle.

In one embodiment, the 3D scanner 200 may be inserted into the oral cavity of the subject 20 to scan the oral cavity in a non-contact manner, thereby acquiring scan data on the oral cavity structure.

For example, scan data for the oral structure may represent an image including a tooth region consisting of at least one tooth, a gingival region, an artificial structure insertable into the oral cavity, or the like. Here, the artificial structure may include an orthodontic device including a bracket and a wire, a prosthesis such as an inlay and a veneer, an implant, a denture, an orthodontic auxiliary device inserted into the oral cavity, a splint, and the like.

For example, the 3D scanner 200 may emit light into the oral cavity of the subject 20 using a light source (or a projector). As a specific example, the 3D scanner 200 may emit light to at least a portion of the oral cavity, such as a tooth region or a gum region, and receive light reflected from the oral cavity of the subject 20 through a camera (or an image sensor). As another example, the 3D scanner 200 may acquire scan data on the intraoral structure by scanning a diagnostic model of the oral cavity. If the diagnostic model of the oral cavity is a diagnostic model modeled after the intraoral structure of the subject 20, the scan data for the diagnostic model of the oral cavity may be scan data on the intraoral structure of the subject 20. For convenience of explanation, the following description will assume a case in which scan data on the intraoral structure is acquired by scanning the oral cavity of the subject 20, but the present disclosure is not limited thereto.

In an embodiment, the 3D scanner 200 may acquire 2D scan data of the intraoral structure of the object 20 on the basis of information received through the camera. Here, the 2D scan data of the intraoral structure may represent a 2D image of the intraoral structure.

For example, 2D scan data for the intraoral structure of the subject 20 may represent a 2D image including a tooth region, a gingival region, an artificial structure, a tongue, and the like, inside the oral cavity of the subject 20.

In an embodiment, the 2D scan data of the oral cavity structure acquired from the 3D scanner 200 may be transmitted to an electronic apparatus 100 through a wired or wireless communication network.

For example, the electronic apparatus 100 may be a computer apparatus or a portable communication apparatus. The electronic apparatus 100 may generate 3D scan data of the intraoral structure, which represents the intraoral structure in three dimensions, based on the 2D scan data of the intraoral structure received from the 3D scanner 200. As a specific example, the electronic apparatus 100 may generate 3D scan data of the intraoral structure by three-dimensionally modeling the intraoral structure on the basis of the received 2D scan data for the intraoral structure.

In an embodiment, the 3D scanner 200 may scan the intraoral structure of the subject 20 to acquire 2D scan data for the intraoral structure, and generate 3D scan data for the intraoral structure on the basis of the 2D scan data. That is, the 3D scanner 200 may generate 3D scan data for the intraoral structure and transmit same to the electronic apparatus 100.

In an embodiment, the electronic apparatus 100 may be connected to a cloud server (not shown) or a database (not shown).

For example, the electronic apparatus 100 may transmit 2D scan data or 3D scan data of the intraoral structure of the subject 200 to the cloud server or database, and the cloud server or database may store 2D scan data or 3D scan data of the intraoral structure of the subject 20 received from the electronic apparatus 100.

Although the 3D scanner 200 was described above with a focus on a handheld scanner, the present disclosure may also be applied to a table scanner that is fixed to a specific location and used. That is, the method proposed in the present disclosure may also be implemented by a table scanner. The table scanner may generate 3D scan data for a diagnostic model of the oral cavity by scanning the diagnostic model of the oral cavity. Since the light source (or projector) and camera of the table scanner are fixed, the user 10 may scan the diagnostic model of the oral cavity while moving the diagnostic model of the oral cavity.

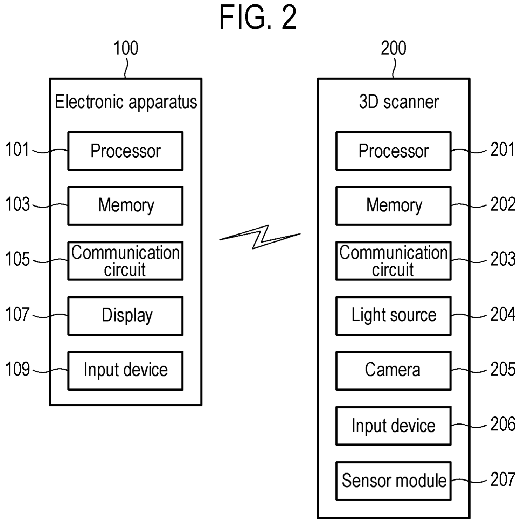

FIG. 2 is a block diagram of an electronic apparatus 100 and a 3D scanner 200 according to an exemplary embodiment of the present disclosure.

In an embodiment, the electronic apparatus 100 and the 3D scanner 200 may be communicatively connected to each other through a wired or wireless communication network, and may transmit and receive various data to and from each other.

In an embodiment, the 3D scanner 200 may include at least one of a processor 201, a memory 202, a communication circuit 203, a light source 204, a camera 205, an input device 206, and a sensor module 207. At least one of components included in the 3D scanner 200 may be omitted or another component may be added to the 3D scanner 200. Additionally or alternatively, some of the components may be integrated and implemented, or implemented as a singular or plural entities. At least some of the components in the 3D scanner 200 may be connected to each other through a bus, a general purpose input/output (GPIO), a serial peripheral interface (SPI) or a mobile industry processor interface (MIPI), and transmit and receive data and/or a signal.

In an embodiment, the processor 201 of the 3D scanner 200 corresponds to a component capable of performing calculation or data processing for control or communication of each component of the 3D scanner 200 and may be operatively connected to the components of the 3D scanner 200. The processor 201 may load a command or data received from other components of the 3D scanner 200 into the memory 202, process the command or data stored in the memory 202, and store result data.

In an embodiment, the memory 202 of the 3D scanner 200 may store at least one instruction for the operation of the processor 201.

In an embodiment, the communication circuit 203 of the 3D scanner 200 may establish a wired or wireless communication channel with an external apparatus including the electronic apparatus 100 and transmit and receive various data to and from the external apparatus.

For example, the communication circuit 203 may include at least one port for connection with the external apparatus through a wired cable to communicate with the external apparatus by wire. In this case, the communication circuit 203 may perform communication with the external apparatus wiredly connected through at least one port.

For example, the communication circuit 203 may include a cellular communication module and may be configured to be connected to a cellular network (e.g., 3G, LTE, 5G, Wibro or WiMAX).

For example, the communication circuit 203 may include a short-range communication module to transmit and receive data to and from the external apparatus including the electronic apparatus 100 using short-range communication (e.g., Wi-Fi, BLE (Bluetooth, Bluetooth Low Energy), or UWB).

For example, the communication circuit 203 may include a non-contact communication module for non-contact communication. As a specific example, the non-contact communication may include at least one non-contact type proximity communication technology such as near field communication (NFC) communication, radio frequency identification (RFID) communication, or magnetic secure transmission (MST) communication.

In an embodiment, the light source 204 of the 3D scanner 200 may emit light toward the inside of the oral cavity of the subject 20. For example, the light emitted from the light source 204 may be structured light having a predetermined pattern. As a specific example, the predetermined pattern may be a stripe pattern in which straight lines of different colors appear continuously. The pattern of the structured light may be generated using a pattern mask or a digital micro-mirror device (DMD), but is not limited thereto.

In an embodiment, the camera 205 of the 3D scanner 200 may acquire 2D scan data of the intraoral structure of the subject 20 by receiving reflected light reflected by the intraoral structure of the subject 20.

For example, the camera 205 may include a left camera corresponding to the left eye field of view and a right camera corresponding to the right eye field of view to construct 3D scan data according to an optical triangulation method. For example, the camera 205 may include at least one image sensor such as a CCD sensor or a CMOS sensor.

In an embodiment, the input device 206 of the 3D scanner 200 may receive a user input for controlling the 3D scanner 200. For example, the input device 206 may include at least one of a button for receiving a push operation of the user 10, a touch panel for detecting a touch of the user 10, or a voice recognition device including a microphone. For example, the user 10 may control scanning start or stop by using the input device 206.

In an embodiment, the sensor module 207 of the 3D scanner 200 may detect an operation state of the 3D scanner 200 or an external environmental state (e.g., a user's motion), and generate an electrical signal corresponding to the detected state. For example, the sensor module 207 may include at least one of a gyro sensor, an acceleration sensor, a gesture sensor, a proximity sensor, or an infrared sensor.

For example, the user 10 may control scanning start or stop by using the sensor module 207. As a specific example, when the user 10 holds the 3D scanner 200 in his or her hand and moves same, the 3D scanner 200 may control, when an angular velocity measured through the sensor module 207 exceeds a predetermined threshold value, the processor 201 to start a scanning operation.

In an embodiment, in response to receiving a user input to initiate a scan through the input device 206 of the 3D scanner 200 or the input device 109 of the electronic apparatus 100, the 3D scanner 200 may initiate a scan.

In an embodiment, the 3D scanner 200 may start scanning on the basis of processing by the processor 201 of the 3D scanner 200 or the processor 101 of the electronic apparatus 100.

In an embodiment, when the user 10 scans the intraoral structure of the subject 20 by using the 3D scanner 200, the 3D scanner 200 may generate 2D scan data of the intraoral structure of the subject 20 and transmit the 2D scan data of the intraoral structure of the subject 20 to the electronic apparatus 100 in real time.

For example, the electronic apparatus 100 may display a 2D image of the intraoral structure represented by the received 2D scan data of the intraoral structure of the subject 20 through a display 107.

For example, the electronic apparatus 100 may generate 3D scan data of the intraoral structure of the subject 20 on the basis of the 2D scan data of the intraoral structure of the subject 20. In addition, the electronic apparatus 100 may display a 3D image of the intraoral structure represented by the received 3D scan data of the intraoral structure of the subject 20 through the display 107. The electronic apparatus 100 may also display the process of generating the 3D scan data of the intraoral structure in real time through the display 107.

For example, the electronic apparatus 100 may generate data on an intraoral fixture that may be mounted on at least a portion of the intraoral structure on the basis of the 3D scan data on the intraoral structure of the subject 20. Here, the intraoral fixture may be a fixture (device) that is designed for various purposes such as fixing teeth of a patient, orthodontic treatment, and tooth protection and may be mounted inside the oral cavity of the patient. For example, the intraoral fixture may be referred to as a tooth fixing device for fixing teeth of the patient, an orthodontic treatment device for correcting teeth of the patient, a tooth protection device for protecting teeth of the patient, or the like. Meanwhile, the present disclosure is not limited thereto, and the intraoral fixture is a term encompassing various types of devices that may be mounted inside the oral cavity, and even if referred to as a term having the same or similar meaning thereto, embodiments of the present disclosure may be applied to such devices. The data on the intraoral fixture may represent an image of the shape of the intraoral fixture.

The electronic apparatus 100 may display an image of the shape of the intraoral fixture through the display 107. The electronic apparatus 100 may also display the process of generating data for the intraoral fixture in real time through the display 107. In addition, the electronic apparatus 100 may display an image of the shape of the intraoral fixture through a wiredly or wirelessly connected external display.

In an embodiment, the electronic apparatus 100 may include at least one of one or more processors 101, one or more memories 103, a communication circuit 105, a display 107, and an input device 109. At least one of components included in the electronic apparatus 100 may be omitted or another component may be added to the electronic apparatus 100. Additionally or alternatively, some of the components may be integrated and implemented, or implemented as a singular or plural entities. At least some of the components within the electronic apparatus 100 may be connected to each other via a bus, GPIO, SPI, MIPI, or the like, and exchange signals or data with each other.

In an embodiment, the one or more processors 101 of the electronic apparatus 100 may be configured to perform operations or data processing related to control or communication of each component of the electronic apparatus 100.

For example, the one or more processors 101 may be operatively connected to components of the electronic apparatus 100. The one or more processors 101 may load a command or data received from other components of the electronic apparatus 100 into the one or more memories 103, process the command or data stored in the one or more memories 103, and store result data.

In an embodiment, the one or more memories 103 of the electronic apparatus 100 may store at least one instruction for an operation of the one or more processors 101. For example, the one or more memories 103 may store data (e.g., 2D scan data or 3D scan data of the intraoral structure) received from the 3D scanner 200.

In an embodiment, the communication circuit 105 of the electronic apparatus 100 may establish a wired or wireless communication channel with an external apparatus including at least one of the 3D scanner 200, a cloud server (not shown), a printer, and the like, and may transmit and receive various data to and from the external device. For example, the communication circuit 105 may include at least one port for connection with the external apparatus through a wired cable to communicate with the external apparatus by wire. In this case, the communication circuit 105 may perform communication with the external apparatus wiredly connected through at least one port.

For example, the communication circuit 105 may include a cellular communication module and may be configured to be connected to a cellular network (e.g., 3G, LTE, 5G, Wibro or WiMAX).

For example, the communication circuit 105 may include a short-range communication module to transmit and receive data to and from an external device including at least one of the 3D scanner 200, a cloud server (not shown), a printer, and the like, by using short-range communication (e.g., Wi-Fi, BLE, UWB).

For example, the communication circuit 105 may include a non-contact communication module for non-contact communication. As a specific example, the non-contact communication may include at least one non-contact type proximity communication technology such as NFC communication, RFID communication, or MST communication.

In an embodiment, the display 107 of the electronic apparatus 100 may display various screens on the basis of the control of the processor 101. For example, the processor 101 of the electronic apparatus 100 may control components of the electronic apparatus 100 to display 2D scan data or 3D scan data of the intraoral structure of the subject 20 through the display 107. For example, the processor 101 may control components of the electronic apparatus 100 to display, through the display 107, data about an intraoral fixture that may be mounted on at least a portion of the oral cavity of the subject 20.

For example, a specific application execution screen may be displayed on the display 107 of the electronic apparatus 100, and 2D scan data or 3D scan data for the intraoral structure may be displayed on the execution screen. In addition, data for the intraoral fixture may be displayed on the execution screen. Here, a web browser or application for executing a specific application may be installed on the electronic apparatus 100. The user 10 may edit, save, and delete 2D scan data or 3D scan data for the intraoral structure displayed on the display 107 by using the input device 109. In addition, the user 10 may edit, save, and delete data for the intraoral fixture displayed on the display 107, by using the input device 109.

In an embodiment, the input device 109 of the electronic apparatus 100 may receive commands or data to be used in a component (e.g., the one or more processors 101) of the electronic apparatus 100 from an external source (e.g., the user 10) of the electronic apparatus 100. For example, the input device 109 may be combined with the display 107 and implemented in a form of a touch sensor panel capable of recognizing contact or proximity of various external objects.

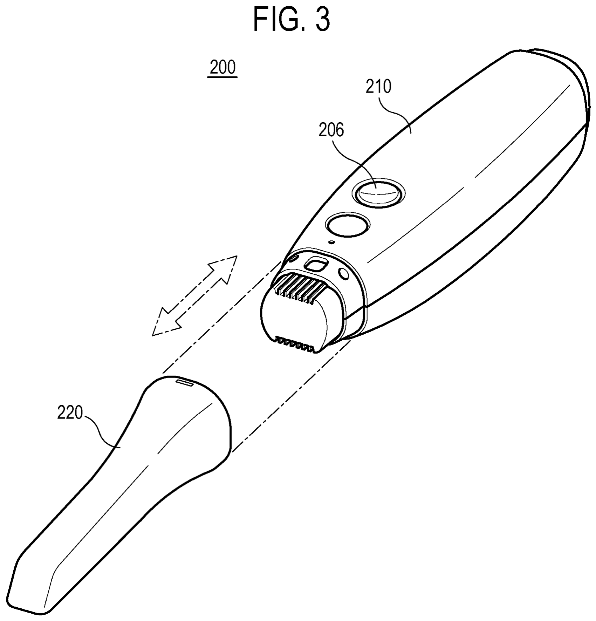

FIG. 3 is a perspective diagram of a 3D scanner 200 according to an exemplary embodiment of the present disclosure.

In an embodiment, the 3D scanner 200 may include a body 210 and a probe tip 220. For example, the body 210 may have a shape that is easy for the user 10 to hold by hand. For example, the probe tip 220 may have a shape that is easy to be inserted into and drawn from the oral cavity of the subject 20. For example, the body 210 may be coupled to and separated from the probe tip 220. For example, the body 210 may have the components of the 3D scanner 200 described in FIG. 2A disposed therein.

For example, an opening may be formed at one end of the body 210 so that light output from the light source 204 may be emitted into the oral cavity of the subject 20. The light emitted through the opening may be reflected by the intraoral structure of the subject 20 and introduced again through the opening. The reflected light entering through the opening may be captured by the camera 205 to generate 2D scan data on the intraoral structure of the subject 20.

For example, the user 10 may start scanning by using the input device 206 (e.g., a button) of the 3D scanner 200. As a specific example, when the user 10 touches or presses the input device 206, light may be emitted from the light source 204 to the subject 20.

In an embodiment, the user 10 may scan the intraoral structure of the subject 20 while moving the 3D scanner 200, and the 3D scanner 200 may acquire 2D scan data of the intraoral structure of the subject 20.

In an embodiment, the user may scan a diagnostic model of the inside of the oral cavity of the subject 20 while moving the 3D scanner 200, and may acquire 2D scan data for the diagnostic model in the process. Here, the 2D scan data for the diagnostic model may represent an image of the shape of the intraoral structure.

For example, the 2D scan data for the intraoral structure may represent a 2D image for an area including the front teeth of the subject 20, a 2D image for an area including the molars of the subject 20, and the like.

For example, the 3D scanner 200 may transmit the acquired 2D scan data to the electronic apparatus 100. In this case, the electronic apparatus 100 may generate 3D scan data for the intraoral structure on the basis of the received 2D scan data for the intraoral structure. Here, the 3D scan data for the intraoral structure may include at least one of maxillary scan data, mandibular scan data, or bimaxillary (occlusion) scan data of the intraoral structure.

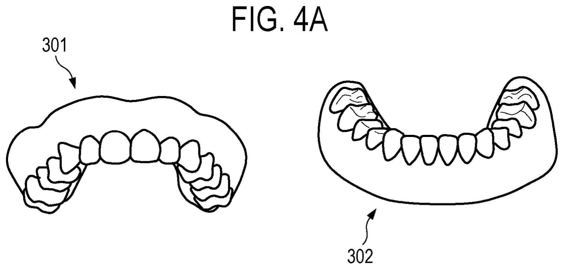

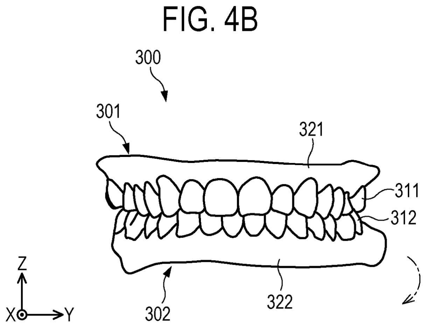

FIGS. 4A and 4B are diagrams illustrating maxillary scan data 301, mandibular scan data 302, and bimaxillary scan data 300 of an oral cavity structure according to an exemplary embodiment of the present disclosure.

In an embodiment, the 3D scan data for the surface of the subject 20 may include at least one of maxillary scan data 301, mandibular scan data 302, or bimaxillary scan data 300. Here, the surface of the subject 20 may include the surface of the intraoral structure of the subject 20.

In an embodiment, the maxillary scan data 301 may be 3D scan data of the maxilla surface generated on the basis of 2D scan data acquired as a result of scanning the maxilla surface within the oral cavity. For example, the maxillary scan data 301 may represent a tooth region 311 and a gingival region 321 within the maxilla. For example, on the basis of the maxillary scan data 301, data for an intraoral fixture that may be mounted on the maxilla within the oral cavity may be generated.

In an embodiment, the mandibular scan data 302 may be 3D scan data of the mandible surface generated on the basis of 2D scan data acquired as a result of scanning the mandible surface within the oral cavity. For example, the mandibular scan data 302 may represent a tooth region 312 and a gingival region 322 within the mandible. For example, on the basis of the mandibular scan data 302, data for an intraoral fixture that may be mounted on the mandible within the oral cavity may be generated.

In an embodiment, the bimaxillary scan data 300 may be 3D scan data of the bimaxillary surface generated on the basis of 2D scan data acquired as a result of scanning the maxilla surface and the mandible surface within the oral cavity. Alternatively, the bimaxillary scan data 300 may be generated on the basis of the maxillary scan data 301 and the mandibular scan data 302. That is, the bimaxillary scan data 300 may be scan data representing an image generated by synthesizing an image represented by the maxillary scan data 301 and an image represented by the mandibular scan data 302. For example, on the basis of the bimaxillary scan data 300, data on an intraoral fixture that may be simultaneously mounted on the maxilla and mandible within the oral cavity may be generated.

Hereinafter, the description of a data processing method proposed in the present disclosure will continue assuming that data for an intraoral fixture is generated on the basis of the mandibular scan data 302. Meanwhile, this is only for convenience of explanation, and the embodiments of the present disclosure are not limited thereto. That is, the description below may be equally applied even when data for an intraoral fixture is generated based on the maxillary scan data 301 or the bimaxillary scan data 300.

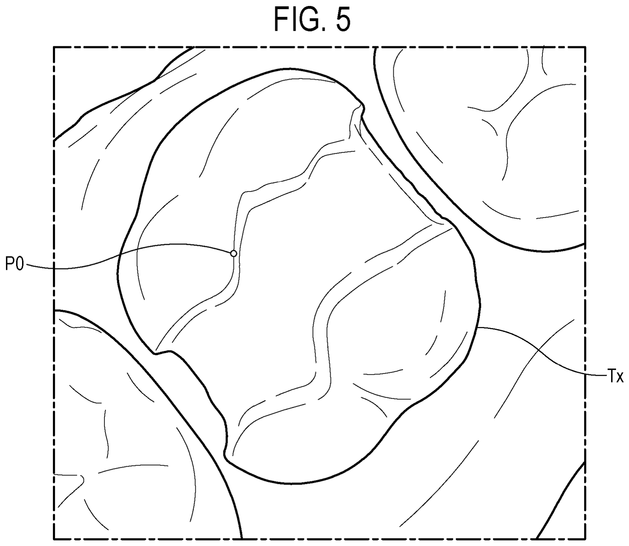

FIG. 5 is a diagram illustrating a process of identifying an initial point for margin line generation according to an exemplary embodiment of the present disclosure.

Specifically, FIG. 5 illustrates a first tooth model (Tx) on an exemplary 3D oral cavity model, and the 3D oral cavity model may be displayed through the display 107 of the electronic apparatus 100 or an external display. The 3D oral cavity model may be an example of the 3D scan data described above. The first tooth model (Tx) may represent some of the tooth data constituting the bimaxillary scan data 300 described above.

The electronic device 100 may identify an initial point PO on the oral cavity model. The initial point P0 may be utilized as an initial position for generating a margin line to be described below. The electronic apparatus 100 according to an embodiment may identify the initial point PO by receiving an input specifying the initial point P0 from the outside. The input specifying the initial point P0 received from the outside may be a point specified by an external user, or may be a point appropriate for generating a margin line derived through an external computing device or the like. In addition, the electronic apparatus 100 may identify the initial point P0 by deriving an appropriate point through internal calculations through the configuration of the processor 101 and the memory 103 or the like. For example, the initial point P0 may be derived through a computing operation utilizing artificial intelligence (AI), and the initial point PO may be identified based on curvature information of the oral cavity model.

The initial point PO according to an embodiment may be located on a region where a margin line is to be formed on the first tooth model Tx. Here, the margin line may refer to a boundary line formed between the aforementioned artificial structures such as prostheses and teeth. The margin line may indicate a boundary between the inner surface and the outer surface of a prosthesis for a specific tooth of a target oral cavity expressed by the 3D oral cavity model. The outer surface of the prosthesis may be a part that is exposed when the prosthesis is actually inserted into the target oral cavity, and the inner surface of the prosthesis may be a part that is not exposed when the prosthesis is actually inserted into the target oral cavity. Here, the margin line may refer to a line spaced a certain distance apart from the boundary line between the artificial structure and the tooth, considering the thickness or step of the artificial structures such as prostheses. The initial point PO according to an embodiment may be located on a region where a margin line is to be formed on the first tooth model Tx, for example, a region where the artificial structure is to be inserted, and has a large curvature on the tooth surface.

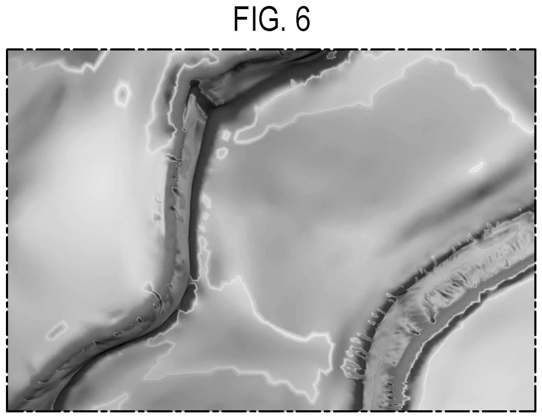

The electronic apparatus 100 may identify the initial point P0 and determine the search direction for generating the margin line. The method by which the electronic apparatus 100 determines the search direction and determines the search points will be described in detail below with reference to FIG. 6, etc.

FIG. 6 is a diagram illustrating curvature information for a 3D oral cavity model according to an exemplary embodiment of the present disclosure.

Specifically, FIG. 6 is a diagram illustrating curvature information on a part of the first tooth model Tx of FIG. 5. The curvature information may be derived from data regarding the 3D oral cavity model, or may be input as curvature information corresponding to the 3D oral cavity model from the outside. The curvature information regarding the 3D oral cavity model may include principal curvature values for points forming a curved surface of the oral cavity model. The curvature information according to an embodiment may be displayed through the display 107 of the electronic apparatus 100 or an external display, but the curvature information does not necessarily need to be displayed, and the process of displaying the curvature information through the display may be omitted. In addition, the electronic apparatus 100 may display the curvature information on the display in response to a request received from the outside. The displaying of the curvature information according to an embodiment may represent curvature distributions distributed on the curved surface of the oral cavity model in different colors or shades.

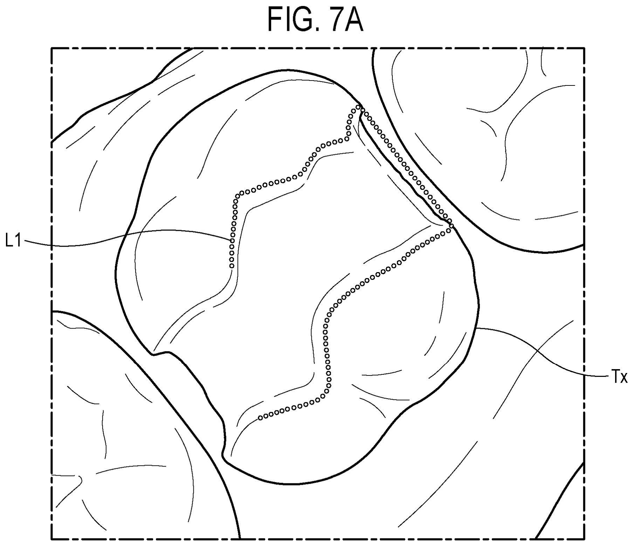

The electronic apparatus 100 may determine a search direction from the identified initial point PO on the basis of curvature information for the 3D oral cavity model. For example, the electronic apparatus 100 may identify points of the oral cavity model adjacent to the initial point PO and identify points that satisfy a predetermined curvature reference among the points of the oral cavity model adjacent to the initial point P0. The electronic apparatus 100 according to an embodiment may determine, as the search direction, a direction from the initial point PO toward a point having the largest curvature magnitude among the points of the oral cavity model adjacent to the initial point P0. In addition, the electronic apparatus 100 according to an embodiment may determine, as the search direction, a direction from the initial point PO to an opposite point having the largest curvature magnitude among the points of the oral cavity model adjacent to the initial point P0. Details related to the determination of the search direction and the search of the search points will be described below with reference to FIGS. 7A to 7C.

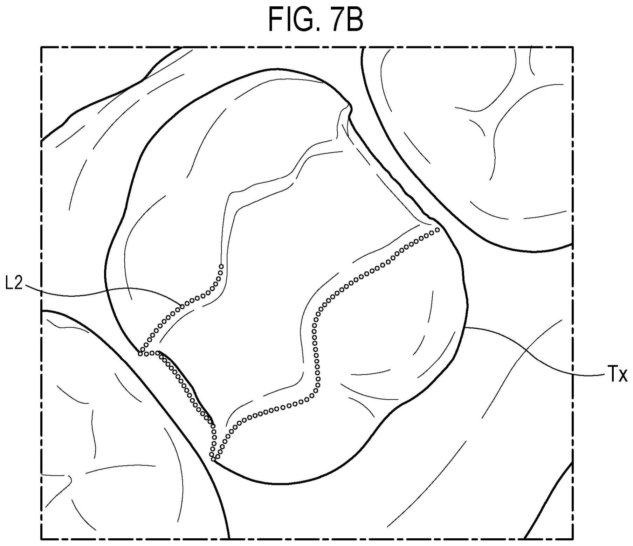

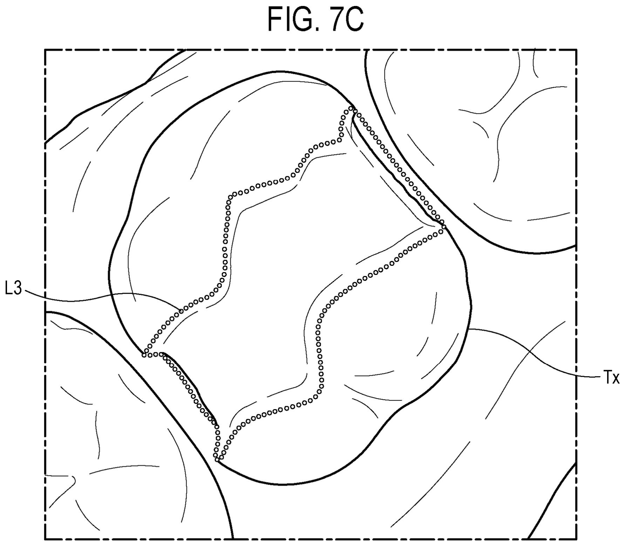

FIGS. 7A, 7B, and 7C are diagrams illustrating multiple first search points, multiple second search points, and an initial closed path according to an exemplary embodiment of the present disclosure.

Specifically, FIG. 7A illustrates multiple first search points positioned along a first search direction according to an exemplary embodiment of the present disclosure, FIG. 7B illustrates multiple second search points positioned along a second search direction according to an exemplary embodiment of the present disclosure, and FIG. 7C illustrates an initial closed path according to an exemplary embodiment of the present disclosure. Hereinafter, FIGS. 7A, 7B, and 7C will be described with reference to the drawings described above, and a description overlapping the above description will be omitted.

Referring to FIGS. 7A and 7B, the electronic apparatus 100 according to an embodiment may determine, as a first search direction, a direction from the initial point PO toward a point having the largest curvature magnitude among the points of the oral cavity model adjacent to the initial point P0. In addition, the electronic apparatus 100 according to an embodiment may determine, as a second search direction, a direction from the initial point PO to an opposite point having the largest curvature magnitude among the points of the oral cavity model adjacent to the initial point P0. In other words, the electronic apparatus 100 may determine a direction reverse or opposite to the first search direction as the second search direction. Alternatively, the electronic apparatus 100 may determine, as the second search direction, a direction from the initial point PO to a point having the second largest curvature magnitude among the points of the oral cavity model adjacent to the initial point P0. In addition, the electronic apparatus 100 according to an embodiment may determine, as the second search direction, a direction from the initial point PO toward a point having the largest curvature magnitude among points located in a direction facing a point having the largest curvature magnitude among points of the oral cavity model adjacent to the initial point P0. The first search direction and the second search direction may be directions moving in different directions from the initial point P0. The method of determining the first search direction and the second search direction is exemplary, and other suitable methods for generating a margin line may also be used.

The electronic apparatus 100 according to an embodiment may sequentially determine multiple first search points L1 on the 3D oral cavity model based on curvature information and the first search direction. For example, the electronic apparatus 100 may determine each of the multiple first search points on the basis of the curvature information and most recently determined one or more first search points.

Specifically, the electronic apparatus 100 according to an embodiment may update the first search direction on the basis of most recently determined one or more first search points. For example, the electronic apparatus 100 may update the first search direction on the basis of six previous points from the currently searched point. The six previous points are merely exemplary values, and the search direction update may be performed with reference to a number (different from six) of points appropriate for the update of the first search direction. In addition, the update of the first search direction may be performed after a sufficient number of first search points are initially determined. For example, in updating the first search direction, if the electronic apparatus 100 determines the first search point on the basis of the six previous points, the first search points may be determined on the basis of the initial search direction which has been derived from the initial point PO and curvature information, until the first six search points are initially determined. After determining six or more first search points, the electronic apparatus 100 may update the first search points on the basis of most recently determined six first search points. It is noted that the number of most recently determined first search points described above is exemplary, and the search direction may be updated on the basis of a different number of search points.

The electronic apparatus 100 may determine a next first search point on the basis of the curvature information and the updated first search direction. The electronic apparatus 100 may determine multiple first search points L1 while sequentially determining the first search points. The electronic apparatus 100 may generate a margin line indicating a closed path on the 3D oral cavity model on the basis of the multiple first search points L1.

The electronic apparatus 100 according to an embodiment may sequentially determine multiple second search points L2 on the 3D oral cavity model based on curvature information and the second search direction. For example, the electronic apparatus 100 may determine each of the multiple second search points on the basis of the curvature information and one or more most recently determined second search points. As described above, the second search direction may be a reverse direction of the first search direction.

Specifically, the electronic apparatus 100 according to an embodiment may update the second search direction on the basis of most recently determined two or more second search points. For example, the electronic apparatus 100 may update the second search direction on the basis of six previous points from the currently searched point. The six previous points are exemplary values, and the search direction update may be performed with reference to a number of points appropriate for updating the second search direction. The electronic apparatus 100 may determine a next second search point on the basis of the curvature information and the updated second search direction. The electronic apparatus 100 may determine multiple second search points L2 while sequentially determining the second search points.

The electronic apparatus 100 may terminate the operation of sequentially determining search points (e.g., the first search point, the second search point, or the like) when a predetermined termination condition is satisfied. Specifically, the electronic apparatus 100 may terminate the operation of sequentially determining search points in response to determining that the predetermined termination condition is satisfied. In this case, the determination that the termination condition is satisfied may be based on various conditions. Exemplary termination conditions may be based on at least one of the number of multiple search points, the length of a path including at least some of the multiple search points, the absence of a point in a current search direction from a previously determined search point, or the absence of a point adjacent to a previously determined search point in the current search direction and satisfying a predetermined curvature reference. For example, when the number of consecutive search points reaches a certain number (e.g., 200), the electronic apparatus 100 may determine that sufficient search points have been found and terminate the operation of sequentially determining search points. As another example, the electronic apparatus 100 may terminate the operation of sequentially determining search points when a path indicated by the search points reaches a certain length (e.g., 20 mm), assuming that sufficient search points have been found. In addition, the electronic apparatus 100 may terminate the operation of sequentially determining search points when there is no point matching the current search direction or when there is no point satisfying the curvature reference among the adjacent points. The above-described conditions are exemplary, and different conditions may be used depending on the case.

Referring to FIG. 7C, the electronic apparatus 100 may determine an initial closed path L3 including multiple first search points L1 and multiple second search points L2. For example, the electronic apparatus 100 may remove overlapping points among the multiple first search points L1 and the multiple second search points L2. In addition, the electronic apparatus 100 may generate an initial closed path L3 by connecting a search point having a minimum distance from a corresponding search point to the corresponding search point with respect to each of the multiple first search points L1 and the multiple second search points L2. However, this is exemplary, and the electronic apparatus 100 may also determine the initial closed path L3 on the basis of additional search points in addition to the first search point L1 and the multiple second search points L2. Additionally, the electronic apparatus 100 may generate the initial closed path L3 on the basis of only one of the multiple first search points L1 or the multiple second search points L2. Additionally, the electronic apparatus 100 may determine whether the initial closed path L3 is twisted or folded, and in response to determining that the initial closed path L3 is twisted or folded, may remove the twisted or folded portion of the initial closed path L3.

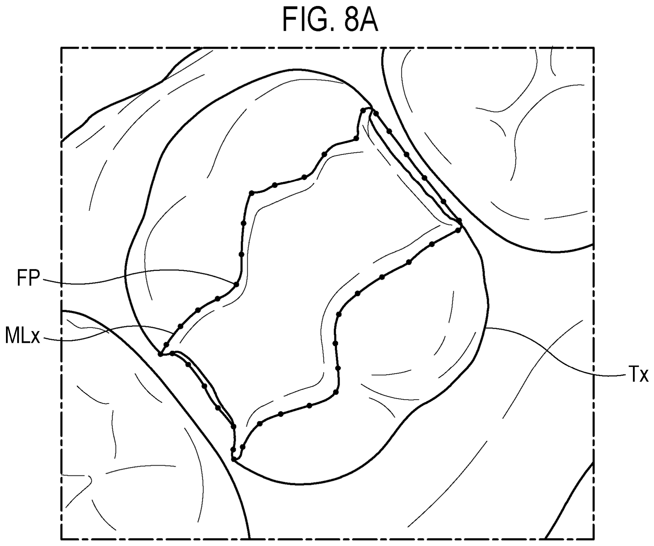

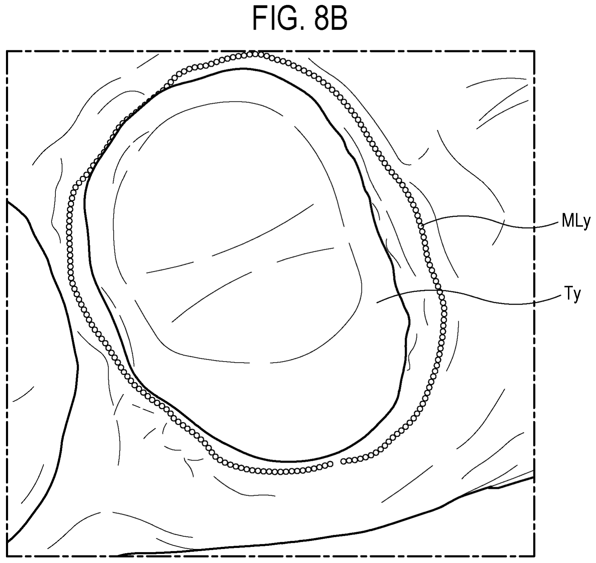

The electronic apparatus 100 may generate a margin line on the basis of the initial closed path L3. For example, the electronic apparatus 100 may generate a margin line by performing a post-processing operation on the initial closed path L3. A specific method for finally generating the margin line will be described in detail below with reference to FIGS. 8A and 8B.

FIGS. 8A and 8B are diagrams illustrating generation of a margin line according to an exemplary embodiment of the present disclosure.

Specifically, FIG. 8A illustrates the generation of a margin line MLx corresponding to a first tooth model Tx according to an exemplary embodiment of the present disclosure, and FIG. 8B illustrates the generation of a margin line MLy corresponding to a second tooth model Ty according to an exemplary embodiment of the present disclosure. Hereinafter, FIGS. 8A and 8B will be described with reference to the drawings described above, and a description overlapping with the above description will be omitted.

Referring to FIG. 8A, the electronic apparatus 100 may generate the margin line MLx based on the initial closed path L3 illustrated in FIGS. 7A to 7C. For example, the electronic apparatus 100 may generate the margin line MLx by performing a post-processing operation, such as line smoothing, with respect to the initial closed path L3 at least once. In addition, the electronic apparatus 100 may generate the margin line MLx by performing projection on a 3D oral cavity model with respect to the initial closed path L3 at least once. According to an embodiment, the electronic apparatus 100 may generate the margin line MLx configured as a smooth curve by performing line smoothing for the initial closed path L3 and projection on the 3D oral cavity model several times. The generated margin line MLx may be utilized to generate a prosthesis corresponding to the first tooth model Tx in the future. In addition, the electronic apparatus 100 may perform an operation of appropriately modifying the margin line for use in manufacturing a prosthesis according to a real-time modification method to be described below.

Referring to FIG. 8B, the electronic apparatus 100 may generate the margin line MLy corresponding to a second tooth model Ty that is different from the first tooth model Tx. The margin line MLx and the margin line MLy are margin lines generated corresponding to exemplary embodiments, and it is noted that the electronic apparatus 100 may generate margin lines for different tooth models, and a margin line different from the margin line MLx and the margin line MLy may be generated.

According to the above-described embodiment, the margin line of the teeth may be automatically and quickly generated on the basis of the 3D image of the oral cavity. In addition, the margin line operation for producing a prosthesis may be performed quickly and efficiently through the automatic generation of the margin line.

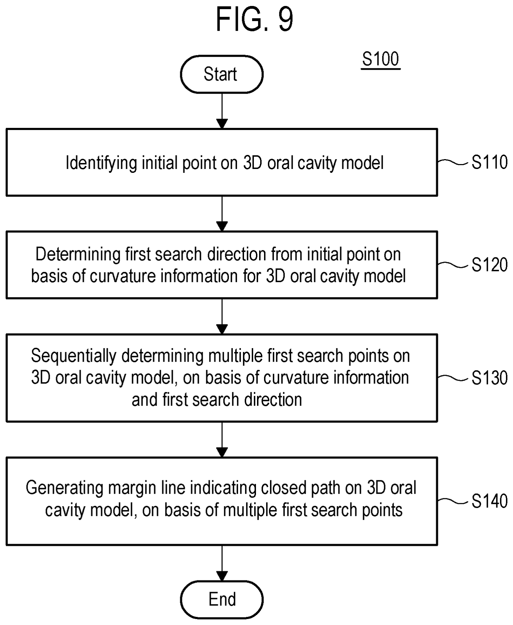

FIG. 9 is a flowchart illustrating a method for generating a margin line according to an exemplary embodiment of the present disclosure.

Referring to FIG. 9, the electronic apparatus 100 may perform a method S100 for generating a margin line including at least some of multiple operations (S110 to S140). Hereinafter, FIG. 9 will be described with reference to the drawings described above, and a description overlapping with the above description will be omitted.

In operation S110, the electronic apparatus 100 may identify an initial point P0 on a 3D oral cavity model. The electronic apparatus 100 according to an embodiment may identify the initial point PO by receiving an input specifying the initial point PO from the outside. The input specifying the initial point PO received from the outside may be a point specified by an external user, or may be a point appropriate for generating a margin line derived through an external computing device or the like. In addition, the electronic apparatus 100 may identify the initial point PO by deriving an appropriate point through internal calculations through the configuration of the processor 101 and the memory 103 or the like.

In operation S120, the electronic apparatus 100 may determine a first search direction from the initial point on the basis of curvature information for the 3D oral cavity model. For example, the electronic apparatus 100 may identify points of the oral cavity model adjacent to the initial point P0 and identify points that satisfy a predetermined curvature reference among the points of the oral cavity model adjacent to the initial point P0. The electronic apparatus 100 according to an embodiment may determine, as the search direction, a direction from the initial point P0 toward a point having the largest curvature magnitude among the points of the oral cavity model adjacent to the initial point P0.

In operation S130, the electronic apparatus 100 may sequentially determine multiple first search points L1 on the 3D oral cavity model based on curvature information and the first search direction. For example, the electronic apparatus 100 may determine each of the multiple first search points on the basis of the curvature information and most recently determined one or more first search points. The electronic apparatus 100 may determine multiple first search points L1 while sequentially determining the first search points.

In addition, the electronic apparatus 100 may terminate the operation of sequentially determining search points (e.g., the first search point, the second search point, or the like) when a predetermined termination condition is satisfied. Specifically, the electronic apparatus 100 may terminate the operation of sequentially determining search points in response to determining that the predetermined termination condition is satisfied.

In operation S140, the electronic apparatus 100 may generate a margin line MLx indicating a closed path on a 3D oral cavity model on the basis of multiple first search points L1. For example, the electronic apparatus 100 may perform a post-processing operation with respect to the multiple first search points L1 to generate a margin line MLx indicating a closed path. Any content overlapping with the above-described content related to the generation of the margin line will be omitted.

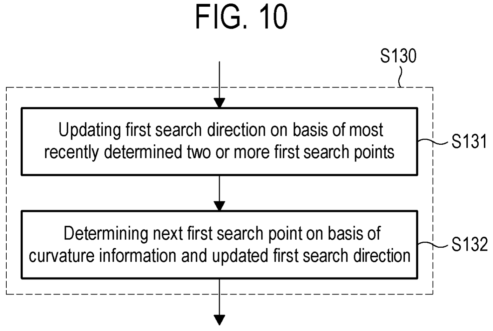

FIG. 10 is a flowchart illustrating a method for sequentially determining search points according to an exemplary embodiment of the present disclosure.

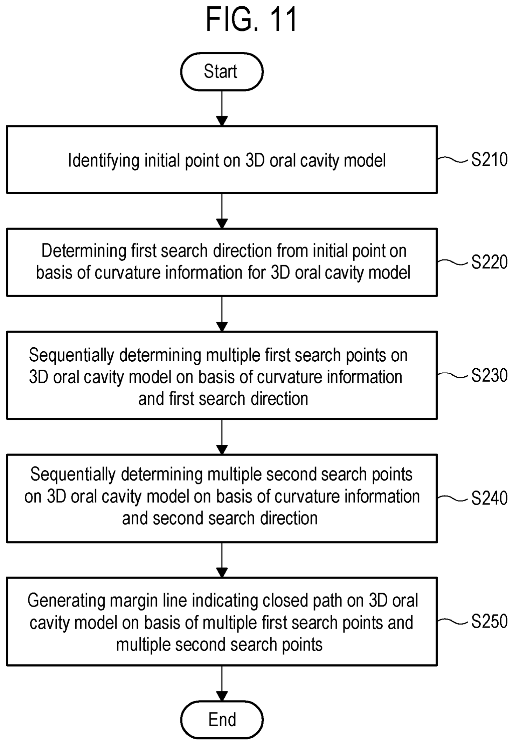

Referring to FIG. 10, the electronic apparatus 100 may perform operation S130 described above in FIG. 9, including multiple operations (S131 to S132). Hereinafter, FIG. 11 will be described with reference to the drawings described above, and a description overlapping with the above description will be omitted.

In operation S131, the electronic apparatus 100 may update the first search direction on the basis of most recently determined two or more first search points. For example, the electronic apparatus 100 may update the first search direction on the basis of six previous points from the currently searched point. The six previous points are exemplary values, and the search direction update may be performed with reference to a number of points appropriate for updating the first search direction.

In operation S132, the electronic apparatus 100 may determine a next first search point on the basis of the curvature information and the updated first search direction. The electronic apparatus 100 may determine multiple first search points L1 while sequentially determining the first search points.

According to the above-described embodiment, the margin line of the teeth may be automatically and quickly generated on the basis of the 3D image of the oral cavity. In addition, the margin line operation for producing a prosthesis may be performed quickly and efficiently through the automatic generation of the margin line.

FIG. 11 is a flowchart illustrating a method for generating a margin line according to an exemplary embodiment of the present disclosure.

Referring to FIG. 11, the electronic apparatus 100 may perform a method of generating a margin line by performing multiple operations (S210 to S250). Hereinafter, FIG. 11 will be described with reference to the drawings described above, and a description overlapping with the above description will be omitted.

In operation S210, the electronic apparatus 100 may identify an initial point PO on a 3D oral cavity model. Operation 210 may be an example of operation S110 in FIG. 9.

In operation S220, the electronic apparatus 100 may determine a first search direction from the initial point on the basis of curvature information for the 3D oral cavity model. For example, the electronic apparatus 100 may identify points of the oral cavity model adjacent to the initial point P0 and identify points that satisfy a predetermined curvature reference among the points of the oral cavity model adjacent to the initial point P0. Operation S220 may be an example of operation S120 in FIG. 9.

In operation S230, the electronic apparatus 100 may sequentially determine multiple first search points L1 on the 3D oral cavity model based on curvature information and the first search direction. The electronic apparatus 100 may determine multiple first search points L1 while sequentially determining the first search points. Operation S230 may be an example of operation S130 in FIG. 9.

In operation S240, the electronic apparatus 100 may sequentially determine multiple second search points L2 on the 3D oral cavity model based on curvature information and the second search direction. For example, the electronic apparatus 100 may determine each of the multiple second search points on the basis of the curvature information and one or more most recently determined second search points.

Specifically, the electronic apparatus 100 according to an embodiment may update the second search direction on the basis of most recently determined two or more second search points. For example, the electronic apparatus 100 may update the second search direction on the basis of six previous points from the currently searched point. The six previous points are exemplary values, and the search direction update may be performed with reference to a number of points appropriate for updating the second search direction. The electronic apparatus 100 may determine a next second search point on the basis of the curvature information and the updated second search direction. The electronic apparatus 100 may determine multiple second search points L2 while sequentially determining the second search points.

The electronic apparatus 100 may terminate the operation of sequentially determining search points (e.g., the first search point, the second search point, or the like) when a predetermined termination condition is satisfied. Specifically, the electronic apparatus 100 may terminate the operation of sequentially determining search points in response to determining that the predetermined termination condition is satisfied.

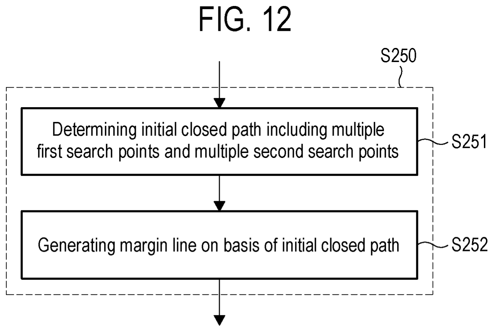

In operation S250, the electronic apparatus 100 may generate a margin line MLx indicating a closed path on a 3D oral cavity model on the basis of the multiple first search points L1 and the multiple second search points L2. A specific example of operation S250 will be described in detail in FIG. 12 below.

FIG. 12 is a flowchart illustrating a method for generating a margin line indicating a closed path according to an exemplary embodiment of the present disclosure.

Referring to FIG. 12, the electronic apparatus 100 may perform operation S250 described above in FIG. 11, including multiple operations (S251 to S252). Hereinafter, FIG. 12 will be described with reference to the drawings described above, and a description overlapping with the above description will be omitted.

In operation S251, the electronic apparatus 100 may determine an initial closed path L3 including the multiple first search points L1 and the multiple second search points L2. For example, the electronic apparatus 100 may remove overlapping points among the multiple first search points L1 and the multiple second search points L2. In addition, the electronic apparatus 100 may generate an initial closed path L3 by connecting a search point having a minimum distance from a corresponding search point to the corresponding search point with respect to each of the multiple first search points L1 and the multiple second search points L2.

In operation S252, the electronic apparatus 100 may generate a margin line MLx on the basis of the initial closed path L3. For example, the electronic apparatus 100 may generate the margin line MLx by performing a post-processing operation, such as line smoothing, with respect to the initial closed path L3 at least once. In addition, the electronic apparatus 100 may generate the margin line MLx by performing projection on a 3D oral cavity model with respect to the initial closed path L3 at least once. According to an embodiment, the electronic apparatus 100 may generate the margin line MLx configured as a smooth curve by performing line smoothing for the initial closed path L3 and projection on the 3D oral cavity model several times.

According to the above-described embodiment, the margin line of the teeth may be automatically and quickly generated on the basis of the 3D image of the oral cavity. In addition, the margin line operation for producing a prosthesis may be performed quickly and efficiently through the automatic generation of the margin line.

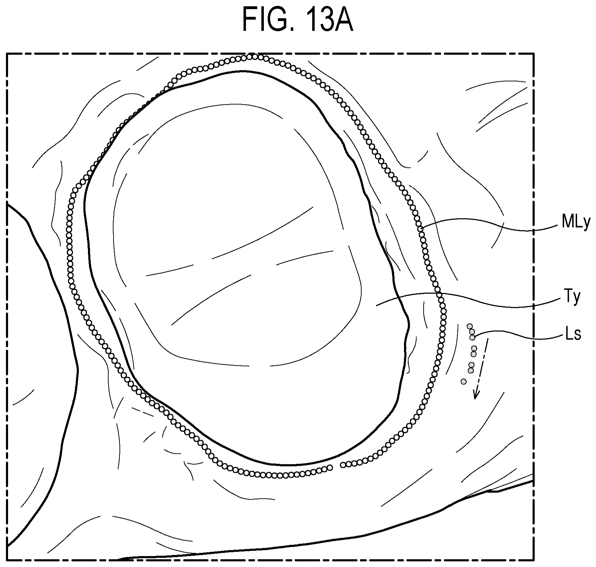

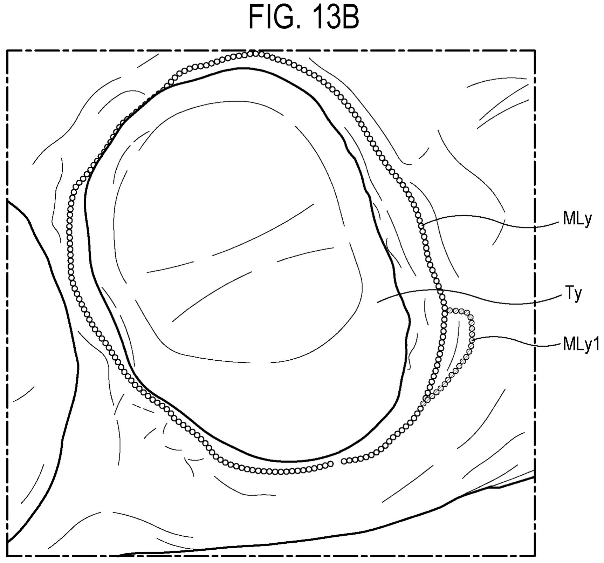

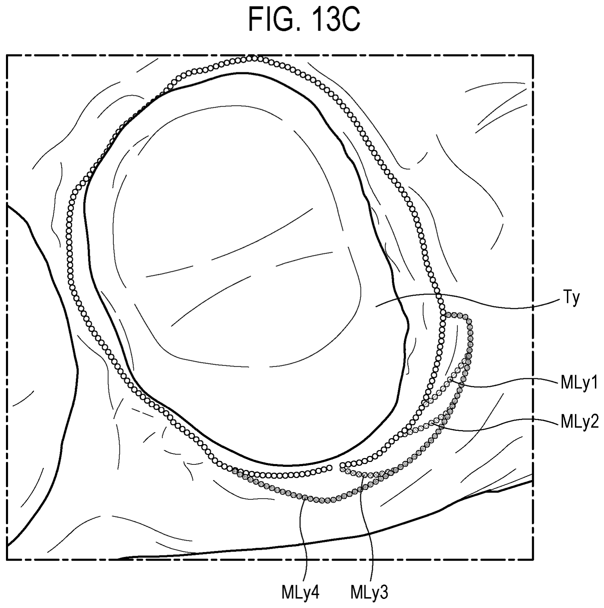

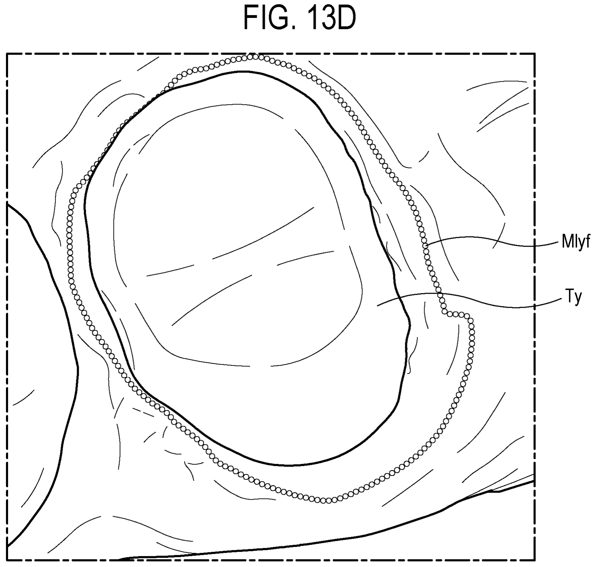

FIGS. 13A, 13B, 13C, and 13D are diagrams illustrating a method for modifying a margin line according to an exemplary embodiment of the present disclosure.

Specifically, FIGS. 13A to 13D illustrate a method of modifying a margin line MLy generated corresponding to a second tooth model Ty according to an exemplary embodiment of the present disclosure. Hereinafter, FIGS. 13A and 13D will be described with reference to the drawings described above, and a description overlapping with the above description will be omitted.

Referring to FIGS. 13A to 13D, the electronic apparatus 100 may modify the generated margin line MLy in real time. Prior to modification, the electronic apparatus 100 according to an embodiment may display a 3D oral cavity model and the margin line MLy through the display 107 or an external display. Thereafter, the electronic apparatus 100 may receive an input for entering a modification mode for the margin line MLy. For example, the input for entering the modification mode may be a mouse click, a mouse click while pressing a control key of a keyboard, or a touch input for a specific interactive element of the display. This is an exemplary input, and other different inputs may be inputs for entering the modification mode for the margin line MLy. Meanwhile, the input for entering the modification mode may also be used as an input for maintaining the modification mode. For example, while maintaining a mouse click while pressing a control key of a keyboard, the modification mode may be maintained.

The electronic apparatus 100 may initiate the modification mode in which a movement input is continuously received in response to the input for entering the modification mode. The electronic apparatus 100 may release the modification mode according to reception of a release input for the modification mode. The movement input according to an embodiment may be a mouse drag or a touch input of the display. However, this is exemplary, and a different input may be presented as the movement input, and the movement input may be changed according to a configuration.

The electronic apparatus 100 may modify the margin line on the basis of a target line segment Ls indicated by the movement input up to the present, in response to determining that a predetermined repetition condition is satisfied while maintaining the modification mode. For example, the predetermined repetition condition may include a lapse of a predetermined time interval, and may also include reaching a predetermined distance interval of a movement distance indicated by the movement input. If other predetermined repetition conditions are satisfied while maintaining the modification mode, the electronic apparatus 100 may modify the margin line on the basis of the target line segment Ls indicated by the movement input up to the present. Specifically, the electronic apparatus 100 may select one or more points among the points included in the margin line MLy on the basis of the target line segment Ls. Thereafter, the electronic apparatus 100 may move each of the one or more selected points in a direction toward the target line segment Ls.

For example, in order to select one or more points, the electronic apparatus 100 may determine a modification reference point on the margin line MLy on the basis of a last point of the target line segment Ls, and select one or more points on the basis of the modification reference point. Specifically, in order to determine the modification reference point, the electronic apparatus 100 according to an embodiment may project the last point of the target line segment Ls onto a curved surface of the oral cavity of the 3D oral cavity model to derive a first point. Thereafter, the electronic apparatus 100 may project the first point onto the margin line MLy to derive a second point located on the margin line. The electronic apparatus 100 according to an embodiment may determine the second point as the modification reference point, but this is exemplary, and other different points on the margin line MLy may be determined as the modification reference point.

The electronic apparatus 100 according to an embodiment may select points on the margin line MLy within a predetermined distance from the modification reference point when selecting one or more points on the basis of the modification reference point. In this case, the predetermined distance may be designated as a constant value, or may be given as a function of a distance between the modification reference point and the target line segment Ls. For example, the value of the predetermined distance may be determined by a negative correlation in which the closer the distance between the modification reference point and the target line segment Ls, the larger the value of the predetermined distance. In addition, the value of the predetermined distance may be a value that may be changed by an external input.

The electronic apparatus 100 according to an embodiment may move each of the one or more selected points in a direction toward the target line segment Ls. The degree to which each of the selected points is moved in a direction toward the target line segment Ls may be designated as a constant value, or may be given as a function of a distance between the modification reference point and the target line segment Ls. For example, the closer the distance between the modification reference point and the target line segment Ls, the greater the degree to which each of the selected points is moved in a direction toward the target line segment Ls. It should be noted that the above-described embodiments are exemplary cases and are not limited to the above description.

The electronic apparatus 100 may update the displaying of the margin line on the display in response to the margin line MLy. For example, in response to determining that the predetermined repetition condition has been satisfied while maintaining the modification mode, the electronic apparatus 100 may modify the margin line MLy based on a first target line segment indicated by the movement input. The electronic apparatus 100 may modify the margin line MLy on the basis of the first target line segment to generate a first modification margin line MLy1. In addition, the electronic apparatus 100 may update the displaying of the margin line MLy on the display to the first modification margin line MLy1.

A continuous modification mode may be performed even after the first target line segment. On the basis of a second target line segment indicated by a movement input input while maintaining the modification mode, the electronic apparatus 100 may modify the first modification margin line MLy1. The electronic apparatus 100 may modify the first modification margin line MLy1 on the basis of the second target line segment to generate a second modification margin line MLy2. In addition, the electronic apparatus 100 may update the displaying of the first modification margin line MLy1 to displaying of the second modification margin line MLy2 on the display.

A continuous modification mode may be performed even after the second target line segment. On the basis of a third target line segment indicated by a movement input input while maintaining the modification mode, the electronic apparatus 100 may modify the second modification margin line MLy2. The electronic apparatus 100 may modify the second modification margin line MLy2 on the basis of the third target line segment to generate a third modification margin line MLy3. In addition, the electronic apparatus 100 may update the displaying of the second modification margin line MLy2 to displaying of the third modification margin line MLy3 on the display.

A continuous modification mode may be performed even after the third target line segment. On the basis of a fourth target line segment indicated by a movement input input while maintaining the modification mode, the electronic apparatus 100 may modify the third modification margin line MLy3. The electronic apparatus 100 may modify the third modification margin line MLy3 on the basis of the fourth target line segment to generate a fourth modification margin line MLy4. In addition, the electronic apparatus 100 may update the displaying of the third modification margin line MLy3 to displaying of the fourth modification margin line MLy4 on the display.

An interval at which each modification margin line is generated may be determined statically or dynamically so that the user may perceive that the margin line is modified in real time according to his/her movement input while efficiently utilizing resources of the electronic apparatus 100.

The electronic apparatus 100 according to an embodiment may receive an input for exiting from the modification mode for the margin line. The end of an input for entering the modification mode may be used as an input for exiting from the modification mode. The electronic apparatus 100 may release the initiation of the modification mode in response to the input for exiting from the modification mode. Along with the release of the initiation of the modification mode, the electronic apparatus 100 may generate a final modification margin line Mlyf after real-time modification of the margin line is finally completed. That is, the electronic apparatus 100 may generate the final modification margin line MLYf by modifying the margin line MLy several times while maintaining the modification mode. In addition, the electronic apparatus 100 may display the final modification margin line MLyf on the display.

Meanwhile, in the margin line MLy generated through the aforementioned margin line generation process, some of the points constituting the margin line MLx may be fixed, as those of the margin line MLx in FIG. 8A. For example, some of the points constituting the margin line MLy may be fixed at predetermined intervals. For convenience of description, some of the fixed points among the points constituting the margin line MLy will be referred to as "fixed points" below.

The electronic apparatus 100 may, in response to an input for entering the modification mode, additionally fix some of the points constituting the margin line MLy. The fixed points may maintain their positions even while maintaining the modification mode unless the fixed points are released. That is, even when the electronic apparatus 100 enters the modification mode and modifies the margin line MLy, the positions of the fixed points may be maintained and the margin line MLy may be modified.

Here, the electronic apparatus 100 may determine that the above-described modification reference point is located between two specific fixed points. That is, the electronic apparatus 100 may determine two fixed points adjacent to the modification reference point among the fixed points on the basis of the modification reference point. The electronic apparatus 100 according to an embodiment may select one or more points to be moved toward the target line segment Ls among points located between the modification reference point and the two adjacent fixed points. That is, the electronic apparatus 100 may determine that one or more points to be moved toward the target line segment Ls are located between two fixed points on the margin line MLy.

Additionally, the electronic apparatus 100 may unfix some of fixed points on the basis of the movement input received while maintaining the modification mode. For example, in response to determining that the modification reference point has passed one of the fixed points on the margin line MLy according to a change in a last point of the target line segment Ls, the electronic apparatus 100 may unfix the point through which the modification reference point has passed. A position of the unfixed point may be modified according to the movement input while maintaining the modification mode.

According to the above-described embodiment, the margin line of the teeth may be automatically and quickly generated on the basis of the 3D image of the oral cavity. According to the embodiments described above, the user may modify margin lines in real time by performing a minimum of operation while viewing incorrectly generated areas during margin line generation. In addition, the margin line operation for producing a prosthesis may be performed quickly and efficiently through automatic generation and real-time modification of the margin line.

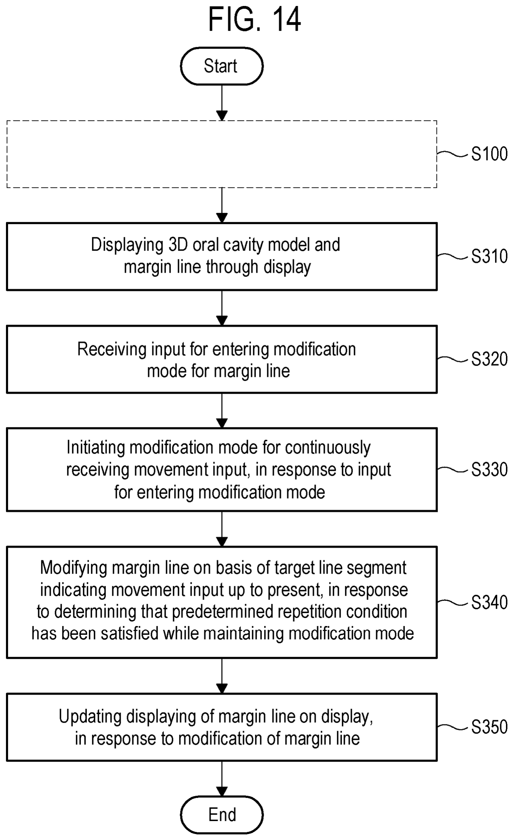

FIG. 14 is a flowchart illustrating a method for modifying a margin line according to an exemplary embodiment of the present disclosure.

Referring to FIG. 14, the electronic apparatus 100 may modify a margin line by performing multiple operations (S310 to S350). Hereinafter, FIG. 14 will be described with reference to the drawings described above, and a description overlapping with the above description will be omitted.

Prior to operation S310, the electronic apparatus 100 may perform the margin line generation method S100 described above. A description overlapping the description in FIG. 9 with respect to the margin line generation method S100 will be omitted. In addition, the electronic apparatus 100 may also receive a margin line generated through an external input without performing the margin line generation method S100.

In operation S310, the electronic apparatus 100 may display the 3D oral cavity model and the margin line MLy through the display 107 or an external display.

In operation S320, the electronic apparatus 100 may receive an input for entering a modification mode for the margin line MLy. For example, the input for entering the modification mode may be a click of a mouse, or a click of a mouse together with pressing a control key. This is an exemplary input, and other different inputs may be inputs for entering the modification mode for the margin line MLy.

In operation S330, the electronic apparatus 100 may initiate the modification mode in which a movement input is continuously received in response to the input for entering the modification mode. The movement input according to an embodiment may be a mouse drag or a touch input of the display.

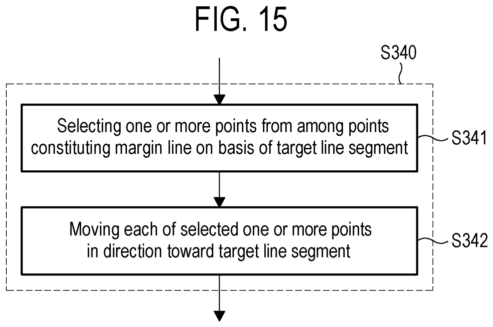

In operation S340, the electronic apparatus 100 may modify the margin line on the basis of a target line segment Ls indicated by the movement input up to the present, in response to determining that a predetermined repetition condition is satisfied while maintaining the modification mode. For example, the predetermined repetition condition may include a lapse of a predetermined time interval, and may also include reaching a predetermined distance interval of a movement distance indicated by the movement input. If other predetermined repetition conditions are satisfied while maintaining the modification mode, the electronic apparatus 100 may modify the margin line on the basis of the target line segment Ls indicated by the movement input up to the present. A specific example of operation S340 will be described in detail in FIG. 15 below.

In operation S350, the electronic apparatus 100 may update the displaying of the margin line on the display in response to modification of the margin line MLy. A description overlapping with that described above in FIGS. 13A to 13D in relation to the modification of the margin line and the update of the display will be omitted.

FIG. 15 is a flowchart illustrating a method for modifying a margin line on the basis of a target line segment according to an exemplary embodiment of the present disclosure.

Referring to FIG. 15, the electronic apparatus 100 may perform operation S340 described above in FIG. 14, including multiple operations (S341 to S342). Hereinafter, FIG. 15 will be described with reference to the drawings described above, and a description overlapping with the above description will be omitted.

In operation S341, the electronic apparatus 100 may select one or more points among the points included in the margin line MLy on the basis of the target line segment Ls. For example, in order to select one or more points, the electronic apparatus 100 may determine a modification reference point on the margin line MLy on the basis of a last point of the target line segment Ls, and select one or more points on the basis of the modification reference point.

In operation S342, the electronic apparatus 100 may move each of the one or more selected points in a direction toward the target line segment Ls.

According to the above-described embodiment, the margin line of the teeth may be automatically and quickly generated on the basis of the 3D image of the oral cavity. According to the embodiments described above, the user may modify margin lines in real time by performing a minimum of operation while viewing incorrectly generated areas during margin line generation. In addition, the margin line operation for producing a prosthesis may be performed quickly and efficiently through automatic generation and real-time modification of the margin line.

As described above, exemplary embodiments have been disclosed with reference to the drawings in the specification. Although specific terms have been used in the specification to describe the embodiments, the terms have been used only for the purpose of explaining the technical idea of the present disclosure and have not been used to limit the meaning or the scope of the present disclosure described in the claims. Therefore, those skilled in the art will understand that various modifications and equivalent other embodiments are possible therefrom. Therefore, the scope of protection of the present disclosure shall be determined by the technical concept of the appended claims.

## Claims

1. A method performed by an electronic apparatus, the method comprising:
identifying an initial point on a three-dimensional oral cavity model;
determining a first search direction from the initial point, based on curvature information about the three-dimensional oral cavity model;
sequentially determining multiple first search points on the three-dimensional oral cavity model, based on the curvature information and the first search direction, each of the multiple first search points being determined based on the curvature information and one or more first search points determined immediately before; and
generating a margin line indicating a closed path on the three-dimensional oral cavity model, based on the multiple first search points.

2. The method of claim 1, wherein the identifying of the initial point comprises receiving an input designating the initial point.

3. The method of claim 1, wherein the sequentially determining of the multiple first search points comprises:
updating the first search direction, based on two or more first search points determined immediately before; and
determining a next first search point, based on the curvature information and the updated first search direction.

4. The method of claim 1, wherein the sequentially determining of the multiple first search points is terminated in response to a determination that a predetermined termination condition is satisfied, and
wherein the determination that the predetermined termination condition is satisfied is based on at least one of the number of the multiple first search points, a length of a path including at least some of the multiple first search points, absence of a point in a current search direction from a first search point determined immediately before, and absence of a point adjacent to a first search point determined immediately before in the current search direction and satisfying a predetermined curvature reference.

5. The method of claim 1, further comprising sequentially determining multiple second search points on the three-dimensional oral cavity model, based on the curvature information and a second search direction from the initial point, the second search direction being opposite to the first search direction, and each of the multiple second search points being determined based on the curvature information and one or more second search points determined immediately before,
wherein the generating of the margin line is additionally based on the multiple second search points.

6. The method of claim 3, wherein the generating of the margin line comprises:
determining an initial closed path including the multiple first search points and the multiple second search points; and
generating the margin line, based on the initial closed path.

7. The method of claim 6, wherein the determining of the initial closed path comprises, for each of the multiple first search points and the multiple second search points, connecting a search point having a minimum distance from a corresponding search point to the corresponding search point.

8. The method of claim 6, wherein the generating of the margin line, based on the initial closed path, comprises generating the margin line by performing smoothing for the closed path and projection on the three-dimensional oral cavity model once or more times.

9. The method of claim 1, wherein the margin line indicates a boundary between an inner surface and an outer surface of a prosthesis for a specific tooth of a target oral cavity expressed by the three-dimensional oral cavity model.

10. The method of claim 1, further comprising displaying the three-dimensional oral cavity model and the margin line through a display.

11. The method of claim 10, further comprising:
receiving an input for entering a modification mode for the margin line;
initiating the modification mode in which a movement input is continuously received, in response to the input for entering the modification mode;
modifying the margin line, based on a target line segment indicated by the movement input up to the present, in response to a determination that a predetermined repetition condition is satisfied while continuing the modification mode; and
updating the displaying of the margin line on the display, in response to modification of the margin line.

12. The method of claim 11, wherein the predetermined repetition condition comprises a lapse of a predetermined time interval.

13. The method of claim 11, wherein the predetermined repetition condition comprises reaching a predetermined distance interval of a movement distance indicated by the movement input.

14. The method of claim 11, wherein the modifying of the margin line comprises:
selecting one or more points among points included in the margin line, based on the target line segment; and
moving each of the one or more selected points in a direction toward the target line segment.

15. The method of claim 14, wherein the selecting of the one or more points comprises:
determining a modification reference point on the margin line, based on a last point of the target line segment; and
selecting the one or more points, based on the modification reference point.

16. The method of claim 15, wherein the selecting of the one or more points, based on the modification reference point, further comprises determining that each of the one or more points is located within a predetermined distance from the modification reference point.

17. The method of claim 15, further comprising fixing some of points included in the margin line, in response to the input for entering the modification mode,
wherein the selecting of the one or more points further comprises:
determining two fixed points among the fixed points, based on the modification reference point, the modification reference point being located between the two fixed points on the margin line; and
determining that the one or more points are located between the two fixed points on the margin line.

18. The method of claim 17, further comprising, in response to determining that the modification reference point has passed one of the fixed points on the margin line according to a change in a last point of the target line segment, unfixing the point through which the modification reference point has passed.

19. An electronic apparatus comprising:
at least one processor; and
at least one memory in which instructions executed by the at least one processor are stored,
wherein the at least one processor is configured to, when the instructions are executed by the at least one processor, execute the method according to one of claims 1 to 18.

20. A non-transitory computer-readable recording medium recording instructions that, when executed by at least one processor, cause the at least one processor to perform operations,
wherein the instructions are configured to cause the at least one processor to execute the method according to one of claims 1 to 18.
